(19) Europäisches Patentamt
European Patent Office
Office européen des brevets

(11) **EP 3 642 618 B1**

(12) **EUROPÄISCHE PATENTSCHRIFT**

(45) Veröffentlichungstag und Bekanntmachung des Hinweises auf die Patenterteilung:
**04.08.2021 Patentblatt 2021/31**

(21) Anmeldenummer: **18734145.8**

(22) Anmeldetag: **18.06.2018**

(51) Int Cl.:
*G01N 33/483* (2006.01)    *C12M 1/42* (2006.01)

(86) Internationale Anmeldenummer:
**PCT/EP2018/066109**

(87) Internationale Veröffentlichungsnummer:
**WO 2018/234238 (27.12.2018 Gazette 2018/52)**

(54) **MULTIELEKTRODENFELD ZUR IMPEDANZMESSUNG AN ADHÄRENTEN ZELLEN**

MULTIELECTRODE ARRAY FOR MEASURING IMPEDANCE OF ADHERENT CELLS

RESEAU DE PLUSIEURS ELECTRODES POUR MESURER L'IMPEDANCE DE CELLULES ADHERENTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.06.2017 DE 102017113748**

(43) Veröffentlichungstag der Anmeldung:
**29.04.2020 Patentblatt 2020/18**

(73) Patentinhaber: **Institut für Bioprozess- und Analysenmesstechnik e.V.**
**37308 Heilbad Heiligenstadt (DE)**

(72) Erfinder: **PLIQUETT, Uwe**
**37308 Heilbad Heiligenstadt (DE)**

(74) Vertreter: **Maikowski & Ninnemann**
**Patentanwälte Partnerschaft mbB**
**Postfach 15 09 20**
**10671 Berlin (DE)**

(56) Entgegenhaltungen:
**CN-U- 203 772 786        US-A1- 2004 152 067**
**US-A1- 2011 121 819**

• **LI XUESI ET AL: "Bioelectric signal detection and stimulation based on transparent oxide microelectrodes", 2016 IEEE 16TH INTERNATIONAL CONFERENCE ON NANOTECHNOLOGY (IEEE-NANO), IEEE, 22. August 2016 (2016-08-22), Seiten 234-237, XP033003843, DOI: 10.1109/NANO.2016.7751539**

**Beschreibung**

**Gebiet der Erfindung**

Die Erfindung liegt auf dem Gebiet der markierungsfreien Beobachtung bzw. Charakterisierung biologischer Objekte auf Basis elektrochemischer Impedanzmessungen. Die Erfindung betrifft insbesondere eine Vorrichtung zur markierungsfreien Charakterisierung und Diskriminierung von Zellen auf Einzelzellniveau sowie Verfahren zur hochkanaligen Impedanzmessung unter Verwendung der erfindungsgemäßen Vorrichtungen. Die Erfindung betrifft weiterhin Anordnungen und ein Verfahren zur schnellen und stromsparenden Impedanzanalytik.

**Hintergrund der Erfindung**

Markierungsfreie Methoden zur Charakterisierung und Diskriminierung von Zellen auf Einzelzellniveau entwickeln sich rasant durch den zunehmenden Bedarf in der Pharmaforschung, im Gesundheitswesen aber auch in der Biotechnologie und in der Nahrungsgüterwirtschaft. Es ist eine wesentliche Stärke der Markierung, bestimmte Zellmerkmale eindeutig und mit hohem Kontrast hervorzuheben. Demgegenüber haben markierungsfreie Methoden oft eine geringe Selektivität. Dieser Nachteil kann durch die gleichzeitige Erfassung multipler Eigenschaften, insbesondere an lebenden Objekten, überwunden werden. Der Vorteil liegt in der Untersuchung der lebenden Objekte unter physiologischen Bedingungen sowie in einer teilweise erheblichen Vereinfachung der experimentellen Prozeduren durch Wegfall der Markierung.

Eine Möglichkeit, solche Untersuchungen an lebenden Objekten durchzuführen, bietet die elektrochemische Impedanzanalytik. Die Erfassung elektrischer Eigenschaften lebender Objekte ist mit modernen Bauelementen mit geringem Aufwand möglich. Daneben ist die Methodik schnell und nicht invasiv. Einmal kontaktiert können Zellen nicht nur elektrisch charakterisiert sondern auch elektrisch manipuliert werden. Letzteres erfolgt durch ein über eine Elektrode appliziertes erhöhtes elektrisches Feld, wodurch Membranstrukturen temporär leitfähig werden. Die elektrische Charakterisierung erfolgt einerseits durch Ableitung aktiv elektrischer Signale wie Aktionspotentiale aber auch Potentialdifferenzen an Grenzschichten, andererseits durch die Messung der elektrischen Impedanz. Während aktive Zellreaktionen wie Aktionspotentiale eine direkte Antwort auf einen depolarisierenden Reiz darstellen, kann die Impedanzmessung Auskunft über die Integrität der Zellmembran, Leitfähigkeit des extrazellulären Mediums sowie die zytosolische Leitfähigkeit geben. Die DE 10 2006 011 345 A1 beispielsweise betrifft eine Vorrichtung zur Stimulation vitaler biologischer Gewebe durch ultrakurze Hochspannungspulse mit dem Ziel, die Membransysteme unsymmetrisch zu permeabilisieren und damit ein elektrisches Potential zu erzeugen, dessen Höhe und zeitlicher Verlauf Auskunft über den energetischen Zustand der Zellen, aber auch ihre unmittelbare weitere Entwicklung geben kann, um damit Gewebe durch ein spezifisches Merkmal zu charakterisieren. Neben der Qualitätsbestimmung von Fleisch unmittelbar nach dem Schlachten lässt sich diese Vorrichtung auch für die Vitalitätsbestimmung dicht gepackter Zellen, beispielsweise in Bioreaktoren oder bei Hochdurchsatzmesstechniken, zur Bestimmung des Zellmembranpotentials einsetzen.

Das zellbasierte Screening gewinnt, beispielsweise im Bereich des Wirkstoff-oder Toxizitäts-Screenings zunehmend an Bedeutung, stellt jedoch auch neue Forderungen an geeignete sensorische Prinzipien, um eine zelluläre Reaktion im Rahmen eines biomedizinischen Experiments möglichst sensitiv messbar zu machen. So werden reine Endpunkt-Bestimmungen derzeit zunehmend durch Echtzeit-Verfahren abgelöst, um die zeitliche Dimension einer Zellreaktion mit ihrem großen bioanalytischen Informationsgehalt nutzen zu können. Voraussetzung für eine kontinuierliche, zeitaufgelöste Beobachtung einer Zellpopulation sind nicht-invasive Methoden, bei denen die Messung selbst ohne Einfluss auf die Zellen bleibt. Impedanzmessungen an zellbedeckten Film-Elektroden (Impedimetrie) sind dafür hervorragend geeignet und seit vielen Jahren in der Literatur beschrieben. Von besonders hoher Akzeptanz ist derzeit das sogenannte electric cellsubstrate impedance sensing (ECIS) (Giaever I., Keese C.R. (1984) Monitoring fibroblast behavior in tissue culture with an applied electric field. Proc Natl Acad Sci USA 81:3761-3764; Giaever I., Keese C.R. (1993) A morphological biosensor for mammalian cells. Nature 366:591-592). Die ECIS-Technik ist die Grundlage der meisten impedimetrischen Verfahren, die derzeit auf dem Markt zur Untersuchung von lebenden Zellen verfügbar sind. Die Grundidee besteht darin, adhärente Zellen auf der Oberfläche von dünnen Goldfilm-Elektroden zu kultivieren, die auf dem Boden eines ansonsten herkömmlichen Zellkulturgefäßes aufgebracht sind und typischerweise einen Flächeninhalt von nur 0,0005 bis 0,01 cm$^2$ besitzen. Als Messsignal dient der elektrische Wechselstromwiderstand (Impedanz) der mit Zellkulturflüssigkeit überschichteten Elektrode, der automatisiert als Funktion der Zeit erfasst wird. Adhärieren Zellen an die Oberfläche der Film-Elektroden, steigt der Wechselstromwiderstand mit zunehmender Belegung der Elektroden an, da die Anwesenheit der (dielektrischen) Zellkörper den Stromfluss zwischen Elektrode und überstehendem Medium erschwert (Rädler, U; Wegener, J. (2009) Impedanzbasiertes Screening adhärenter Zellen. BIOspektrum, 05.09, 15. Jahrgang, S. 535-537).

Bei der Besiedlung von Elektrodenstrukturen kann auch die Zelldichte abgeleitet werden. Zelleigenschaften wie die Morphologie oder die Zellgröße lassen sich mit planaren Elektrodenstrukturen nur bedingt erfassen. Diese Größen können vorteilhaft durch eine mikroskopische Observation erfasst werden. Transparente Elektrodenstrukturen geringer Dichte für eine Kontaktierung von Zellen unter mikroskopische Observation sind Stand der Technik. Hochkanalige Elektrodenfelder scheitern jedoch üblicherweise an der aufwändigen oder technisch nicht realisierbaren Kontaktierung individueller Elektroden. Darüber sind Elektrodenfelder auf CMOS-Basis mit bis zu 1000 Einzelelektroden mit Abmessungen individueller Elektroden im Bereich von 100 μm bekannt, die zwar speziell für die Belange der elektrischen Impedanzmessungen entwickelt wurden, jedoch nicht mikroskopierbar sind. Vorrichtungen und Systeme zur Impedanzmessung sind aus dem Stand der Technik bekannt. WO 2008/072166 A1 offenbart beispielsweise eine Vorrichtung zur Zellanalyse, die eine Sandwichstruktur aufweist. Die offenbarte Vorrichtung umfasst einen Speicherkondensator, der dafür verwendet wird, den Schalter für eine längere Zeit im leitenden Zustand zu halten. Dadurch wird die zugehörige Elektrode länger mit Spannung versorgt. Der Kondensator wird nicht für die Messung eines Impedanzspektrums verwendet, sondern für die Modulation des elektrischen Feldes, dem eine Probe ausgesetzt wird.

WO 2008/072135 A2 betrifft eine Vorrichtung für die Elektroporation. Diese Vorrichtung umfasst eine planare Elektrodenmatrix, deren Oberfläche für die zu untersuchenden Proben zugänglich ist. Es werden verschiedene Ausführungsformen für die Ansteuerung der einzelnen Elektroden offenbart, um ein elektrisches Feld der gewünschten Stärke zu applizieren. Nach Durchführung der Elektroporation können die manipulierten Zellen mit optischen Mitteln untersucht werden oder andere Parameter wie z.B. die Impedanz gemessen werden. WO 2008/072135 A2 offenbart keine Hinweise über die Durchführung dieser nachfolgenden Untersuchungen unter Verwendung eines Kondensators.

US 2017/0038282 A1 offenbart eine elektronische Prüfplatte. Diese umfasst eine Multiwellplatte, wobei die Wells auf der Platte verschiedene Dimensionen aufweisen können. Über mehrere Sensoren können verschiedene Parameter (z.B. Impedanz, pH-Wert, optische und akustische Eigenschaften) einer Probe innerhalb eines Wells bestimmt werden. Die Impedanz wird dabei über drei Elektroden sowohl in x-y-Ebene als auch in z-Richtung gemessen. Bezüglich der Signalverarbeitung werden lediglich Verstärker und Filter vorgeschlagen. Die hier beschriebenen Sensoren für die Impedanzmessung funktionieren klassisch im Frequenzbereich.

US2004/152067 offenbart eine Vorrichtung zur elektrischen Charakterisierung und/oder Manipulation biologischer Zellen, mit einem Multielektrodenfeld aus Glas und Elektroden aus ITO, wobei die Elektroden so gestaltet sind, dass biologische Zellen daran adhärieren können. Den Elektroden sind Schalter zugeordnet, sodass durch Auswahl von Elektroden das Impedanzspektrum der adhärierten biologischen Zellen messbar ist.

Die Impedanzmessung ist in vielen Bereichen eine gut etablierte Methode zur zerstörungsfreien elektrischen Charakterisierung von Materialien. Im lebenswissenschaftlichen Bereich gibt es aber zunehmende Forderungen nach einfachen und stromsparenden Systemen für den Point-of-care-Einsatz sowie für Einwegartikel in Schnelltests. Etablierte Systeme, wie die oben beschriebenen, können diese Forderungen in der Regel nicht erfüllen. Ein weiterer Nachteil ist, dass etablierte Systeme beispielsweise nur bei einer Frequenz arbeiten. Für die Erreichung einer praxisrelevanten Spezifität benötigt man jedoch oft Informationen über einen weiten Frequenzbereich.

## Beschreibung der Erfindung

Die Aufgabe der Erfindung besteht daher darin, die Nachteile des Standes der Technik zu überwinden und ein mikroskopierbares Elektrodenfeld bereitzustellen, das eine für zellbasiertes Screening geeignete Fläche aufweist. Jede individuelle Elektrode soll sowohl die aktiven als auch die passiv-elektrischen Eigenschaften adhärierender Zellen erfassen können und in der Lage sein, diese Zellen elektrisch zu stimulieren. Eine weitere Aufgabe der Erfindung besteht darin, Anordnungen und Verfahren für eine breitbandige Impedanzmessung, die speziell für biologische Materialien geeignet ist, mit einem Frequenzumfang von beispielsweise 6 Dekaden im mHz- bis GHz-Bereich bereitzustellen. Die Aufgabe der Erfindung wird gelöst durch eine Vorrichtung zur elektrischen Charakterisierung und elektrischen Manipulation biologischer Objekte gemäß Anspruch 1, wobei die Vorrichtung ein mikroskopierbares Multielektrodenfeld darstellt. Das Multielektrodenfeld ist vorzugsweise in Spalten und Zeilen orientiert. Das Multielektrodenfeld kann beispielsweise 10 x 10 Elektroden (Spalte x Zeile) oder mehr Elektroden pro Spalte und/oder Reihe aufweisen. Vorzugsweise weist das Multielektrodenfeld mindestens 100 x 100 Elektroden (Spalte x Zeile) oder mehr Elektroden pro Spalte und/oder Reihe auf.

"Elektrische Charakterisierung" im Sinne der Erfindung bedeutet die labelfreie Beobachtung der passiven elektrischen Eigenschaften adhärenter Zellen auf Basis elektrochemischer Impedanzmessungen. Die Zellform ändert sich z. B. infolge eines äußeren Stimulus (Wirkstoffzugabe, Transfektion, oxidativer Stress). Nahezu alle Zellen reagieren auf chemische, biologische oder physikalische Stimuli mit mehr oder weniger deutlichen Änderungen ihrer Zellform. Diese Änderung wird in einer Änderung der gemessenen Impedanz abgebildet. Die gemessene Impedanz ist von der Zellform abhängig. Der impedimetrische readout erlaubt eine kontinuierliche Beobachtung der Zellen über variable Zeiträume von wenigen Sekunden bis zu Wochen. Das zur Messung verwendete elektrische Signal ist nicht-invasiv und durch den Einsatz Multiwell-Formaten kann ein mittlerer bis großer Probendurchsatz erzielt werden, wodurch die erfindungsgemäße Vorrichtung und die erfindungsgemäßen Verfahren beispielsweise für labelfreie, zellbasierte Assays für das Wirkstoff-oder Toxizitäts-Screening geeignet sind.

"Elektrische Manipulation" im Sinne der Erfindung bedeutet die Beeinflussung von aktiv-elektrischen Eigenschaften der adhärierten biologischen Objekte, insbesondere adhärierter Zellen, wie zum Beispiel die Beeinflussung von Aktionspotentialen an Membranen der Zellen oder die Durchführung von Elektroporationen. Elektroporation ist eine Methode, Zellmembranen vorübergehend permeabel (durchlässig) zu machen, um so Stoffe und chemische Moleküle, wie zum Beispiel DNA in prokaryotische Zellen (Transformation) oder eukaryotische Zellen (Transfektion) einzuschleusen.

Die erfindungsgemäße Vorrichtung ist insbesondere dann für das zellbasierte Screening geeignet, wenn das mikroskopierbare Multielektrodenfeld etwa die Fläche einer herkömmlichen Multiwellplatte, wie zum Beispiel einer 384-, 96-, 60- oder 48-Wellplatte, besonders bevorzugt einer 48-Wellplatte aufweist. Weiterhin bevorzugt ist es, wenn mindestens 80%, mehr bevorzugt mindestens 85 %, besonders bevorzugt mindestens 90% der Fläche mit Elektrodenstrukturen im Submillimeter-Bereich abgedeckt sind. Submillimeter-bereich in Bezug auf die Größe der Elektroden bedeutet beispielsweise 30 bis 200 $\mu$m. In Bezug auf die Anzahl der Elektroden pro Well ist es bevorzugt, wenn das Multielektrodenfeld 5 bis 100, vorzugsweise 10 bis 90 oder 15 bis 80, mehr bevorzugt 20 bis 70 oder 25 bis 60, besonders bevorzugt 30 bis 50 oder 30 bis 40 Einzelelektroden pro Well einer Multiwellplatte aufweist.

"Mikroskopierbar" im Sinne der Erfindung bedeutet, dass das Multielektrodenfeld und darauf adhärierte biologische Objekte entweder mittels Auflichtmikroskopie oder mittels Durchlichtmikroskopie untersuchbar sind. Bevorzugt ist ein Multielektrodenfeld, das mittels Durchlichtmikroskopie untersuchbar ist.

Entsprechend werden in Feldern angeordnete Elektroden aus transparenten Materialien (z.B. Indium-Zinn-Oxid (indium tin oxide, ITO) auf einem transparenten Substrat wie Glas, Saphir oder Quarz verwendet. Transparente Materialien im Sinne der Erfindung sind Materialien, die die Fähigkeit besitzen, elektromagnetische Wellen, vorzugsweise mit Wellenlägen im sichtbaren Bereich, hindurchzulassen (Transmission). Der besondere Vorteil der Verwendung solcher Materialien liegt darin, dass die erfindungsgemäßen Multielektrodenfelder lichtdurchlässig sind und die Multielektrodenfelder neben der Impedanzmessung zusätzlich lichtmikroskopischen Untersuchungsmethoden zugänglich sind. Die Adressierung der Einzelelektroden erfolgt in Zeilen und Spalten. Geeignet zur Verwendung in der erfindungsgemäßen Vorrichtung sind zum Beispiel kommerziell erhältliche Multielektrodenfelder. Bevorzugt sind insbesondere Multielektrodenfelder, die dem Aufbau der Backplane eines TFT Displays entsprechen. In einer besonders bevorzugten Ausführungsform umfasst die erfindungsgemäße Vorrichtung Multielektrodenfelder, die aus einer herkömmlichen Backplane eines TFT Displays herausgeschnitten wurden. Neben der notwendigen Transparenz hat dies den Vorteil, dass TFT Displays mittlerweile in sehr großen Größen erhältlich sind und damit das in der erfindungsgemäßen Vorrichtung einzusetzende Multielektrodenfeld an jede erforderliche Größe angepasst werden kann. So ist es beispielsweise völlig problemlos, transparente Multielektrodenfelder herzustellen, die die Größe einer 96- Wellplatte aufweisen. Ein weiterer Vorteil solcher Multielektrodenfelder besteht darin, dass an den Multielektrodenanordnungen die gleichzeitige Parallelmessung der elektrischen Impedanz möglich ist.

Nach dem Herausschneiden des Multielektrodenfeldes aus einer kommerziellen Backplane werden freiliegende Drähte vorzugsweise maskiert und metallisiert, um die störungsfreie Anbindung an eine Interface-Elektronik zu gewährleisten. Weiterhin bevorzugt ist es, wenn das Multielektrodenfeld an eine die Interface-Elektronik tragende Leiterplatte gebondet wird oder direkt auf die Oberfläche eines transparenten Materials, wie beispielsweise Glas, aufgebracht wird.

Erfindungsgemäß ist jede Elektrode mit einem integrierenden Glied und einem Schalter gekoppelt. Das integrierende Glied ist vorzugsweise ein direkt an einer Elektrode integrierter Kondensator. In einer bevorzugten Ausführungsform der Erfindung weist jede Elektrode des Multielektrodenfeldes als integrierendes Glied einen Kondensator auf. Der Schalter ist beispielsweise ein Transistor. In einer bevorzugten Ausführungsform der Erfindung weist jede Elektrode des Multielektrodenfeldes als Schalter einen Transistor auf.

In der erfindungsgemäßen Vorrichtung sind die Elektroden so gestaltet sind, dass an den Elektroden biologische Objekte adhärieren können. Durch Selektion bzw. Auswahl mindestens einer Elektrode ist das Impedanzspektrum des an dieser mindestens einen Elektrode adhärierten biologischen Objektes messbar.

"Adhärieren an den Elektroden" im Sinne der Erfindung schließt sowohl das direkte Adhärieren biologischer Objekte an den Elektroden als auch an isolierenden Schichten, die wahlweise auf den Elektroden aufgebracht sein können, ein. Typischerweise wird das Multielektrodenfeld gemäß der Erfindung unterhalb einer Multiwellplatte positioniert, wobei die Böden der Wells vor Aufbringen der Multiwellplatte auf das Multielektrodenfeld entfernt werden. Dadurch befinden sich Elektroden sowohl direkt in den einzelnen Wells, als auch zwischen den Wells. Die biologischen Objekte adhärieren dann beispielsweise an den am Boden der jeweiligen Wells befindlichen Elektroden und sind der Impedanzmessung zugänglich. Die Elektroden zwischen den Wells erhalten keinen Kontakt zu biologischen Objekten. Bei den Impedanzmessungen werden diese Elektronen passiviert und dienen der Kalibrierung des Gesamtsystems.

Diese Anordnung hat weitere Vorteile. Durch eine gemeinsame Stimulierung aller Elektroden fließt je nach Widerstand des angekoppelten Objektes in jeder Elektrode des Multielektrodenfeldes ein Strom. Dieser Strom ist in dem jeder Elektrode zugeordneten integrierenden Glied integrierbar und anschließend sequentiell über alle Elektroden auslesbar.

Vorzugsweise sind die Elektroden so gestaltet, dass das Impedanzspektrum durch Auswertung der Antwort auf einen Spannungssprung messbar ist. Hierzu können einzeln ausgewählte oder alle Elektroden durch einen Spannungssprung angeregt werden und die Stromantwort kann partiell integriert werden, um die durch das an der entsprechenden Elektrode adhärierte Objekt transportierte Ladung nach einer vorbestimmten Zeit nach Auslösen des Spannungssprungs zu erhalten. Durch Wahl der Länge des Stimulus sowie der Spannung kann die insgesamt transportierte Ladungsmenge und somit die Spannung am Ausgang bestimmt werden. Durch Integration mit unterschiedlich langen Stimuli können entsprechende Punkte auf der Sprungantwort gemessen werden. Alternativ kann das Verfahren auch so gestaltet werden, dass das Impedanzspektrum durch Auswertung der Antwort auf einen Stromsprung messbar ist. Hierzu können einzeln ausgewählte oder alle Elektroden durch einen Stromsprung angeregt werden, wobei die Spannung an der Gegenelektrode und die Ladung auf den Kondensatoren ausgewertet wird.

Die Stimulierung bestimmter biologischer Objekte (Zellen) kann auch mit Licht erfolgen. Demzufolge ist es in einer weiteren Ausführungsform der Erfindung vorgesehen, dass die erfindungsgemäße Vorrichtung anstelle der Backplane eines TFT-Displays die Backplane eines OLED-Displays aufweist, wobei optional einige der LEDs mit Elektroden versehen sein können. Auch eine kombinierte TFT-OLED Backplane ist zur Verwendung als Multielektrodenfeld in der erfindungsgemäßen Vorrichtung geeignet.

Die Auswertung erfolgt vorzugsweise unter Verwendung eines numerischen Algorithmus. Der numerische Algorithmus dient entweder der Auswertung direkt im Zeitbereich durch Anpassen von Exponentialfunktionen mit je einer Zeitkonstante und einer Relaxationsstärke (preexponentieller Faktor) oder der Umrechnung in den Frequenzbereich, wobei das individuelle Impedanzspektrum für jede Elektrode als Ergebnis erhältlich ist.

Die Integration des Signals über vordefinierte Zeitbereiche ist besonders vorteilhaft, weil dadurch ein adaptives Anti-Aliasing-Filter realisierbar ist. Zudem sind je nach gewünschtem Frequenzspektrum Messungen an allen Elektroden (bis zu einigen Millionen) in weniger als eine Sekunde realisierbar. Dies bedeutet einerseits, dass eine Vielzahl von Messungen in sehr kurzer Zeit durchführbar ist, andererseits werden auch dynamische Messungen ermöglicht.

Es ist auch möglich, an den Elektroden über aktiv-elektrische Signale zu integrieren. Hierzu kann der der Stimulus auf Masse gezogen werden. Die Zeitauflösung ergibt sich dann durch unterschiedlich gewählte Integrationszeiten. Für eine hohe Zeitauflösung können entweder einzelne Elektroden oder Gruppen von Elektroden ausgelesen werden.

Damit ist die erfindungsgemäße Vorrichtung besonders vorteilhaft, da sowohl die aktiven als auch die passiv-elektrischen Eigenschaften adhärierender Zellen, und zwar an jeder einzelnen Elektrode, erfassbar sind.

Für die Erzeugung elektrischer Stimuli kann der Auslesekanal vorgespannt werden und dieses Potential über die Selektionslogik auf die Elektroden gelegt werden. Alternativ können einzelne Elektroden selektiert werden und der Ausgang auf Masse gezogen werden und ein gemeinsamer Stimulus für alle Kanäle gleichzeitig angelegt werden. In diesem Fall wird das integrierende Glied kurzgeschlossen und eine Messung ist nicht möglich. Mit dieser Anordnung ist es in vorteilhafter Weise möglich, die adhärierten Zellen, und zwar ebenfalls an jeder einzelnen Elektrode des Multielektrodenfeldes, elektrisch zu stimulieren.

Zusammengefasst ist es mittels der erfindungsgemäßen Vorrichtung möglich, mit jeder individuellen Elektrode des Multielektrodenfeldes sowohl die aktiv- und die passiv-elektrischen Eigenschaften adhärierender Zellen zu erfassen, als auch diese Zellen elektrisch zu stimulieren.

Die erfindungsgemäße Vorrichtung weist eine Anordnung, insbesondere für eine Interface-Elektronik auf. Die Komponenten der Interface-Elektronik für die Verwendung in der erfindungsgemäßen Vorrichtung sind kommerziell erhältlich.

Erfindungsgemäß sind die Komponenten der Interface-Elektronik so gestaltet, dass nachfolgend dargestellte erfindungsgemäße Verfahren in vorteilhafter Weise auszuführen. Diesbezüglich weist die Interface-Elektronik Komponenten zur Applikation eines Spannungs- oder Stromsprungs, zur Integration der gemessenen Signale mit gradueller Abtastung auf. Die Interface-Elektronik weist einen Pufferverstärker auf. Dadurch kann die Antwort auf den Spannungs- oder Stromsprung an das Objekt angepasst werden. Weiterhin wird ein logarithmischer Verstärker zwischen Integrator und Abtaststufe geschaltet. Dies hat den Vorteil, dass der Dynamikbereich, d.h. der Bereich der abtastbaren Frequenz auf mehr als 6 Größenordnungen erweitert werden kann. Der Integrator weist unter anderem einen Integrationskondensator auf. Die Abtaststufe kann beispielsweise eine Sample & Hold Schaltung sein. Zusätzlich weist die Interface-Elektronik einen nachgeschalteten Vollweg-Präzisionsgleichrichter auf. Dadurch ist es möglich, auch die komplementäre Halbwelle zur Auswertung heranzuziehen. Dies hat den Vorteil, dass das Signal-Rausch-Verhältnis verbessert wird. Ebenso können auf diese Wiese geringe Nichtlinearitäten ausgeglichen werden. Von der Sample & Hold - Schaltung werden die Daten dann an einen ADC weitergegeben. Vorzugsweise ist die Interface-Elektronik mit einer Datenverarbeitungsanlage verbunden. Als Datenverarbeitungsanlage ist jede bekannte Datenverarbeitungsanlage geeignet, wie z.B. ein PC, Laptop-Computer, Tablet-PC, Mobiltelefon usw. Die Datenverarbeitungsanlage wirkt hierzu über eine Kommunikationsverbindung, beispielsweise eine serielle oder parallele Schnittstelle, USB-Verbindung, Mini-USB-Verbindung oder WLAN-Verbindung oder Bluetooth-Verbindung, mit der Interface-Elektronik zusammen.

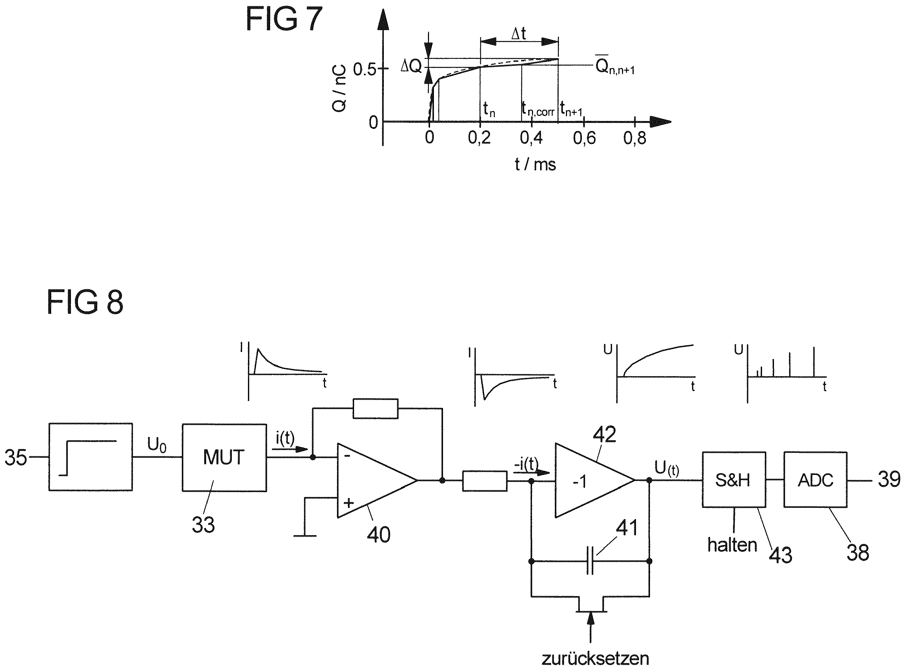

Die Erfindung stellt somit eine Anordnung, insbesondere für eine Interface-Elektronik für die erfindungsgemäße Vorrichtung und das erfindungsgemäße Verfahren bereit, die ein Mittel zur Applikation eines Spannungs- oder Stromsprungs, einen Pufferverstärker, einen Vollweg-Präzisionsgleichrichter, einen logarithmischen Verstärker, einen Integrationskondensator, eine Abtaststufe (Sample & Hold) und einen ADC aufweist. Eine beispielhafte Anordnung einer solchen Interface-Elektronik ist in Figur 8 gezeigt. Diese Anordnung hat, wie bereits erwähnt, den Vorteil, dass der Dynamikbereich, d.h. der Bereich der abtastbaren Frequenz auf mehr als 6 Größenordnungen erweitert werden kann. Ein wesentlicher Nachteil, besonders bei kleinen Signalen (z.B. hohe Impedanz, geringe Messspannung), ist der hohe Einfluss der Ladungsinjektion beim Sample & Hold-Schalter.

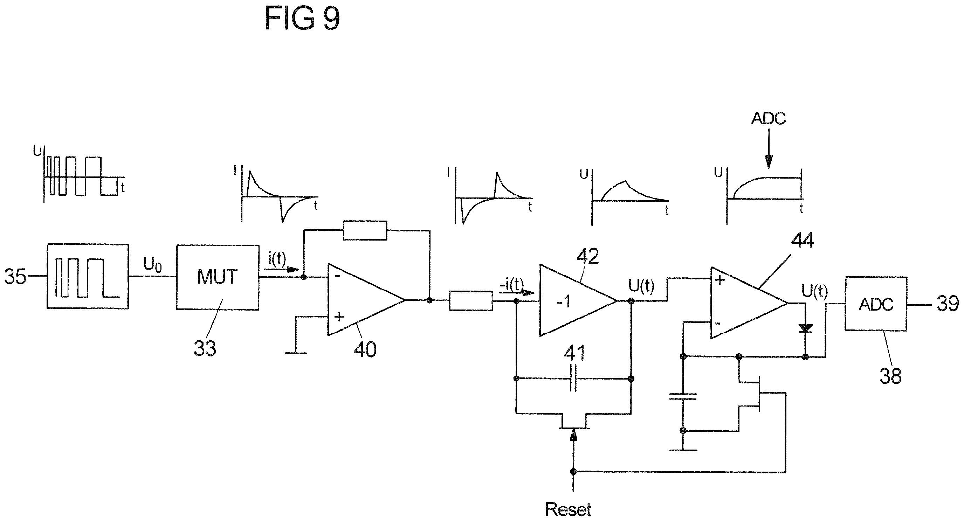

Diese Nachteile können dadurch überwunden werden, in dem die erfindungsgemäße Anordnung anstelle des Abtastglieds (wie z.B. einer Sample & Hold Schaltung) einen Präzisions-Spitzengleichrichter aufweist. Der Hold-Modus wird hier durch Ziehen des Signalpegels auf eine Spannung nahe der negativen Versorgungsspannung erreicht. Die Erfindung stellt somit eine Anordnung zur Verfügung, insbesondere für eine Interface-Elektronik, die ein Mittel zur Applikation eines Spannungs- oder Stromsprungs, einen Pufferverstärker, einen Vollweg-Präzisionsgleichrichter, einen logarithmischen Verstärker, einen Integrationskondensator, einen weiteren Präzisionsgleichrichter und einen ADC aufweist. Eine beispielhafte Anordnung einer solchen Interface-Elektronik ist in Figur 9 gezeigt. Vorteilhaft gegenüber der Variante mit Abtastglied ist die höhere Präzision durch den Verzicht auf eine Abtaststufe, in welcher der Einfluss von Schalttransienten durch höhere Ladeströme und damit durch einen höheren Stromverbrauch minimiert werden muss.

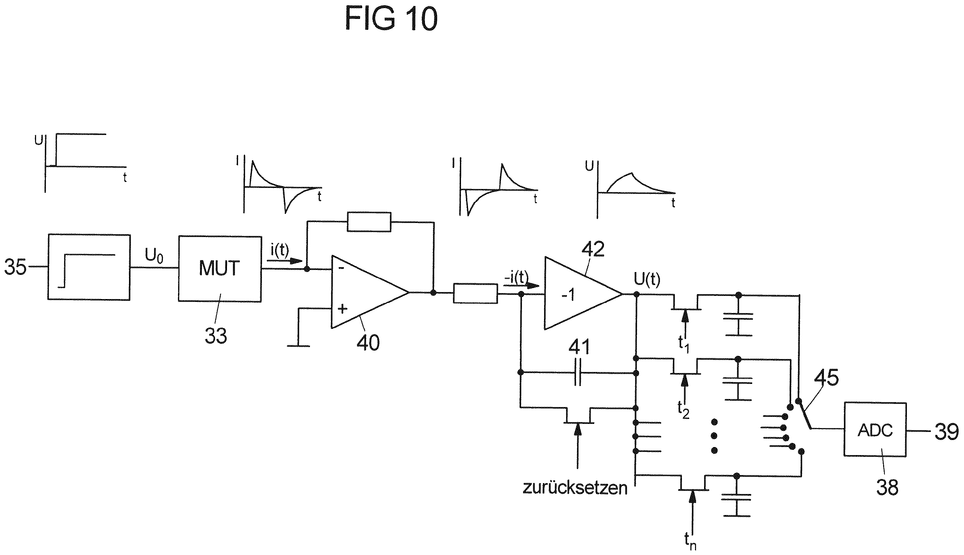

Für kontinuierliche Messungen mit maximaler Wiederholfrequenz müssen die ersten sehr dicht abgetasteten Werte über einen Zeitraum von bis zu $100\,\mu s$ zwischengespeichert werden. In einer weiteren bevorzugten Ausführungsform weist die erfindungsgemäße Anordnung deshalb ein Schieberegister auf. Gesteuert durch das Schieberegister, welches vom logarithmisch eingestellten Abtasttakt geschoben wird, können die einzelnen Werte in einer Kette von Abtastgliedern in individuellen Kondensatoren zwischengespeichert werden. Anstelle der Kette von Abtastgliedern kann auch eine Kette von Präzisionsgleichrichtern verwendet werden. In dieser Ausführungsform kommen insbesondere stromsparende und hochauflösende ADCs (16 bit) zum Einsatz. Durch einen Multiplexer können die Spannungen an den ADC gelegt und umgesetzt werden. Geeignete Multiplexer sind beispielsweise energiesparende RISC-Prozessoren mit bis zu 20 Analogeingängen. Weiterhin können Abtastglieder auch zu Blöcken zusammengefasst werden und durch mehrere ADCs ausgelesen werden. Die Erzeugung der Abtastzeitlage kann durch ein rückgekoppeltes Schieberegister erfolgen, was eine aufwändige Zeitlagensteuerung erübrigt. Diese Anordnung hat den Vorteil, dass sie besonders stromsparend ist und für batteriebetriebene Lösungen einsetzbar ist. Eine beispielhafte Anordnung einer solchen Interface-Elektronik ist in Figur 10 gezeigt.

Mit der erfindungsgemäßen Vorrichtung, die die erfindungsgemäße Anordnung aufweist, ist es in vorteilhafter Weise möglich, eine breitbandige und stromsparende Bestimmung der elektrischen Impedanz eines Materials bzw. biologischen Objekts auf der Grundlage des Relaxationsverhaltens durchzuführen, bei dem die durch die Elektrode fließenden Ladungen integriert werden und ein adaptives Anti-Aliasing-Filter realisiert wird. Die Vorteile der erfindungsgemäßen Vorrichtung liegen darin, dass eine hohe Bandbreite erreicht werden kann und der Stromverbrauch im kontinuierlichen Betrieb sehr niedrig ist und batterieversorgte Anwendungen gestattet. Die Kosten des Gesamtsystems sind gering und ermöglichen Einweganwendungen. Weitere Einzelheiten werden nachfolgend für das erfindungsgemäße Verfahren beschrieben.

Die Erfindung stellt weiterhin Verfahren zur Detektion der aktiv-elektrischen Eigenschaften von biologischen Objekten bereit.

Die zuvor beschriebenen Vorteile und vorteilhaften Ausführungsformen für die erfindungsgemäße Vorrichtung und die erfindungsgemäßen Anordnungen gelten gleichermaßen für das nachfolgend beschriebene Verfahren, so dass auf das zuvor genannte Bezug genommen wird.

In einer bevorzugten Ausführungsform stellt die Erfindung ein Verfahren zur Detektion der passiv-elektrischen Eigenschaften eines biologischen Objekts bereit, wobei das Verfahren folgende Schritte aufweist:

- Erzeugen eines sprunghaften Stromstimulus oder Spannungstimulus, vorzugsweise einzeln an mindestens einer Elektrode oder gleichzeitig an allen Elektroden des Multielektrodenfeldes,
- Erfassung der individuellen Sprungantworten der einzelnen Elektroden in Form des durch die Elektroden fließenden Stroms, wobei eine zeitabhängige Messung des Stroms erfolgt, und
- Erfassung des Frequenzspektrums an einer einzelnen Elektrode.

Das Erzeugen des sprunghaften Stimulus, der die Zellen wegen der geringen Spannung nicht beeinflusst, erfolgt vorzugsweise durch Anregen mit einem Spannungssprung über eine Gegenelektrode. Durch den Spannungssprung wird eine Stromantwort in jeder individuellen Elektrode des Elektrodenfeldes generiert. Vorzugsweise besitzt der sprunghafte Stimulus eine variable Länge. Dies hat den Vorteil, dass unterschiedliche Abtastpunkte der Stromantwort generiert werden.

Alternativ kann das Erzeugen des sprunghaften Stimulus auch durch Anregen einzelner oder aller Elektroden mit einem Stromsprung erfolgen. Durch den Stromsprung wird eine Spannungsantwort generiert. Vorzugsweise besitzt der sprunghafte Stimulus eine variable Länge. Dies hat den Vorteil, dass unterschiedliche Abtastpunkte der Spannungsantwort generiert werden. Bevorzugt ist jedoch das Erzeugen des sprunghaften Stimulus durch Anregen einzelner oder aller Elektroden mit einem Spannungssprung zur Generierung einer Stromantwort.

In einer besonders bevorzugten Ausführungsform der Erfindung wird als sprunghafter Stimulus ein Rechteckpuls erzeugt. Dadurch ist die Ladung der Kondensatoren nach Ablauf eines Rechteckpulses an allen Elektroden erfassbar, bevor der nächste sprunghafte Stimulus erzeugt wird. Wie zuvor beschrieben, ist es besonders vorteilhaft, wenn die Anregung durch Rechteckpulse unterschiedlicher Zeitdauer erfolgt.

Die Lage und Anzahl der Abtastpunkte werden im Wesentlichen durch die Länge (Zeitdauer) des Stimulus bestimmt und die Anzahl steigt vom Erzeugen des sprunghaften Stimulus bis zum Abschalten des Stimulus exponentiell an.

In einem weiteren Verfahrensschritt kann festgestellt werden, an welche Elektroden interessierende Objekten adhäriert sind. Diese Elektroden können dann selektiv mit hohen bzw. erhöhten Messwiederholratenausgelesen werden. Dadurch ist es möglich, die Zeitauflösung einzelner Kanäle zu erhöhen.

In einer weiteren Ausführungsform der Erfindung ist es möglich, auch die aktiv-elektrischen Eigenschaften adhärierter biologischer Objekte zu messen. Dazu werden die Gegenelektrode auf Massepotential gezogen und die durch die Zellen verursachten Ströme, beispielsweise nach einem depolarisierenden Stimulus, gemessen.

In einer weiteren Ausführungsform weist das erfindungsgemäße Verfahren zusätzlich einen Schritt der Generierung eines Selektionsmusters für einen manipulativen Stimulus für die einzelnen Elektroden des Multielektrodenfeldes auf. Dies hat den Vorteil, dass es möglich ist, den manipulativen Stimulus nur an ausgewählten Elektroden, aber nicht an allen Elektroden, zu erzeugen.

Alternativ ist es möglich, alle Elektroden des Multielektrodenfeldes zu selektieren und einen gemeinsamen manipulativen Stimulus zu erzeugen. Dadurch kann das erfindungsgemäße Verfahren und die erfindungsgemäße Vorrichtung auch zur Induktion von Elektroporation oder einem Aktionspotential genutzt werden. Aktionspotentiale können nach Erkennen von Elektroden mit erregbaren Membran von adhärierten biologischen Objekten durch statische Selektion mindestens einer dieser Elektroden und kontinuierlicher Aufzeichnung der Spannung über dem Ladkondensator mit hoher Zeitauflösung gemessen werden.

Die Impedanzspektren werden vorzugsweise mit einem analytischen Algorithmus aus den Abtastpunkten berechnet.

In einer besonders bevorzugten Ausführungsform des erfindungsgemäßen Verfahrens erfolgt die Auswertung im Zeitbereich, wobei jeder Zeitkonstante ein Polarisationsmechanismus zugeordnet wird.

Unter "Polarisationsmechanismus" im Sinne der Erfindung wird eine Methode zur elektrischen Ladungsspeicherung in biologischen Materialien verstanden. Dem Fachmann bekannt sind Polarisationsmechanismen wie Grenzflächenpolarisation an biologischen Membranen, Atompolarisation, ionische Polarisation und Orientierungspolarisation (Ausrichtung von Molekülen in biologischen Systemen.

Die Auswertung von Impedanzspektren im Zeitbereich ist an sich bekannt. Entsprechende Algorithmen wurden beispielsweise beschrieben in Pliquett, U. (2013): Time-domain based impedance measurement: strengths and drawbacks. XV Int. Conf. on Electrical Bio-Impedance & XIV Conf. on Electrical Impedance Tomography, IOP Publishing Journal of Physics: Conference Series 434, 012092 und Pliquett, U. (2015): Adaptive signal sampling for high throughput, broadband impedance spectroscopy. International Journal of Bioelectromagnetism, Vol. 17, No. 1, pp. 20-25.

Abtastschaltungen verwenden hohe Ströme und niederohmige Kopplung, um die Probleme parasitärer Kapazitäten zu minimieren. Diese Lösungen sind im Allgemeinen weder preiswert noch stromsparend. In einer besonders bevorzugten Ausführungsform stellt die Erfindung deshalb ein Verfahren für eine breitbandige Impedanzmessung bereit, das dadurch gekennzeichnet ist, dass anstelle eines Abtastgliedes bzw. einer Kette von Abtastgliedern ein Präzisionsgleichrichter bzw. eine Kette von Präzisionsgleichrichtern verwendet wird. Dies wird durch die konsequente Ausnutzung der Eigenschaften von Sprungantworten biologischer Materialien erreicht. Wesentlich ist dabei, dass die Antwort eine Summe von Exponential- oder Wurzelfunktionen darstellt und damit immer monoton ist.

Die Sprungantwort wird durch partielle Integration konditioniert, wobei die Integrationsdauer vom Sprung bis zum Ende des Stimulus exponentiell verlängert wird und nach jedem Integrationszyklus der Analogwert aufgezeichnet wird. Die graduell abgetastete Sprungantwort wird entweder direkt im Zeitbereich ausgewertet, wobei jeder im Signal auftretenden Zeitkonstante ein Polarisationsmechanismus zugeordnet wird. Eine Transformation in den Frequenzbereich ist, wie oben beschrieben, durch einen analytischen Ansatz möglich, da etablierte Methoden wie schnelle Fouriertransformation für die mit diesem Verfahren generierten Signale nicht funktionieren. Für die Transformation von Systemantworten, die Wurzelfunktionen enthalten, ist ein numerisches Verfahren anzuwenden. Für zeitunkritische Anwendungen wird die partielle Integration mit exponentiell ansteigender Integrationszeit pro Periode eines repetierenden Signals nur einmal ausgeführt, was heißt, dass pro Abtastpunkt eine Periode erforderlich ist. Eine Messgeschwindigkeit am theoretischen Limit kann erreicht werden, wenn nach jeder Integrationszeit die Umsetzung erfolgt, während die Integration fortgeführt wird. in diesem Fall ist die Messung nach einer halben Periode abgeschlossen. Dies erfordert einen schnellen ADC. Um dies zu umgehen, werden in einer Ausführungsform des erfindungsgemäßen Verfahrens schnell auftretende Abtastpunkte nach dem Sprung in mehreren Kondensatoren zwischengespeichert und diese anschließend mit einem langsameren ADC umgesetzt. Ein besonderer Vorteil dieser Anordnung ist die Möglichkeit, unterschiedlich große Kondensatoren zu verwenden, was den Dynamikbereich auch ohne Logarithmierverstärker erheblich erweitert.

In einer besonders bevorzugten Ausführungsform stellt die Erfindung ein Verfahren für eine breitbandige und stromsparende Bestimmung der elektrischen Impedanz eines Materials bzw. biologischen Objekts bereit, bei der in dem Material eine sprunghafte Änderung eines elektrischen Stimulus erzeugt und die resultierende Antwort nach partieller Integration ausgewertet wird, dadurch gekennzeichnet, dass die Zeitlage für die Prozessierung der Sprungantwort sowie für die Generierung der Sprungfrage auch dem gleichen Takt erzeugt werden. Durch partielle Integration kann ein adaptives Anti-Aliasing-Filter realisiert werden. Der Dynamikbereich kann entweder durch geschaltete Kapazitäten eingestellt und/oder durch einen Logarithmierverstärker erweitert werden. In einer bevorzugten Ausführungsform können die die Abtastintervalle dynamisch eingestellt werden. Zur Erhöhung der Präzision kann die Sprungfrage synchron zur Sprungantwort abgetastet werden. Zur Transformation in den Frequenzbereich wird ein spezieller Algorithmus verwendet. In einer bevorzugten Ausführungsform weist der Algorithmus die Schritte auf:

1. Zerlegung der Abtastpunkte in eine Summe bekannter Funktionen (Exponentialfunktionen (einfache Relaxation), Verteilung von Zeitkonstanten (constant phase element), Wurzelfunktion (Elektrodeneigenschaften));
2. Analytische Berechnung der Fouriertransformierten, entweder über eine gesamte Halbwelle oder partiell; oder numerische Berechnung bei Wurzelfunktionen;
3. Zuordnung von Relaxationemechanismen zu Modelleigenschaften; und
4. Optional Transformation der Eigenschaften aus dem Zeitbereich in den Frequenzbereich durch analytische oder auch numerische Algorithmen zur Erzeugung des Impedanzspektrums.

Die Vorteile dieser Variante des erfindungsgemäßen Verfahrens liegen darin, dass eine hohe Bandbreite erreicht werden kann und der Stromverbrauch im kontinuierlichen Betrieb sehr niedrig ist und batterieversorgte Anwendungen gestattet. Die Kosten des Gesamtsystems sind gering und ermöglichen Einweganwendungen.

Besonders bevorzugt ist es, wenn die verschiedenen Ausführungsformen der Erfindung mittels der erfindungsgemäßen Vorrichtung ausgeführt werden.

Die Erfindung wird nachfolgend anhand von zehn Zeichnungen und fünf Ausführungsbeispielen näher erläutert.

Es zeigen:

**Figur 1:** die typische Schaltung **10** der Backplane eines TFT-Displays, wie es aus dem Stand der Technik bekannt ist;

**Figur 2:** ein TFT-Display, in dem die Eigenschaften durch Einführen einer weiteren Masselinie verbessert worden sind;

**Figur 3:** das Prinzip einer Einzelzelle (A) und die typische Geometrie eines Pixels (B);

**Figur 4:** die Geometrie der Backplane eines TFT- LCD;

**Figur 5:** die prinzipielle Ordnung für die Verwendung eines kommerziellen Displays für die elektrische Charakterisierung biologischer Objekte gemäß der Erfindung;

**Figur 6:** das Prinzip der parallelen Messung; und

**Figur 7:** Exponentiell ansteigende Zeitintervalle für die Integration des durch die Elektroden fließenden Stroms.;

**Figur 8:** die Integration des Signals mit gradueller Abtastung;

**Figur 9:** die Verwendung einer Präzisionsgleichrichtung anstatt eines Abtastglieds;

**Figur 10:** eine Schaltung zur parallelen Zwischenspeicherung der Abtastwerte.

## Stand der Technik

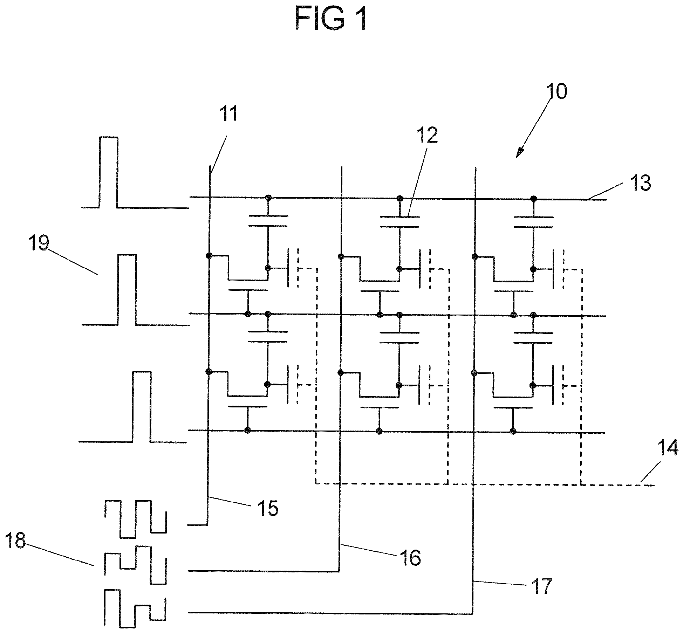

**Figur 1** zeigt die typische Schaltung **10** der Backplane eines TFT-Displays (Quelle: Wikipedia). Das Multielektrodenfeld gemäß der Erfindung weist ein Feld von ITO-Elektroden, jeweils mit integriertem Thin-Film-Transistor (TFT) sowie einem Kondensator auf. Die Architektur entspricht der Backplane der bekannten TFT-Displays. Die Dimensionen dieser konventionellen Elektroden liegen im Bereich von 30-200 $\mu$m, was für die meisten Anwendungen zur Untersuchung von Zellen adäquat ist. Die Größe der Kapazität ist so ausgelegt, dass eine eingeprägte Spannung für wenigstens 30 ms gehalten werden kann. Der Auswahl (bzw. "select")-Draht **13** aktiviert eine gesamte Reihe während die Data-Drähte **11** jeweils eine gesamte Spalte mit den selektierten Kondensatoren verbinden. Dies erfordert einen Multikanal-Multiplexer oder entsprechend viele Analog-Digital-Wandler am Ausgang. In einem einfachen Beispiel werden die Ausgänge sequentiell über ein Schieberegister an einen Entladewiderstand gelegt und der Strom gemessen und gewandelt.

Die transparente Elektrode ist einerseits mit der Source des TFT und andererseits mit dem Speicherkondensator **12** verbunden. Bei einer nicht selektierten Spalte liegt das Gate auf -5V, während 20V für die Auswahl angelegt werden. Die Daten für die selektierte Spalte werden als Analogspannung parallel für die gesamte Zeile am Drain der Transistoren appliziert.

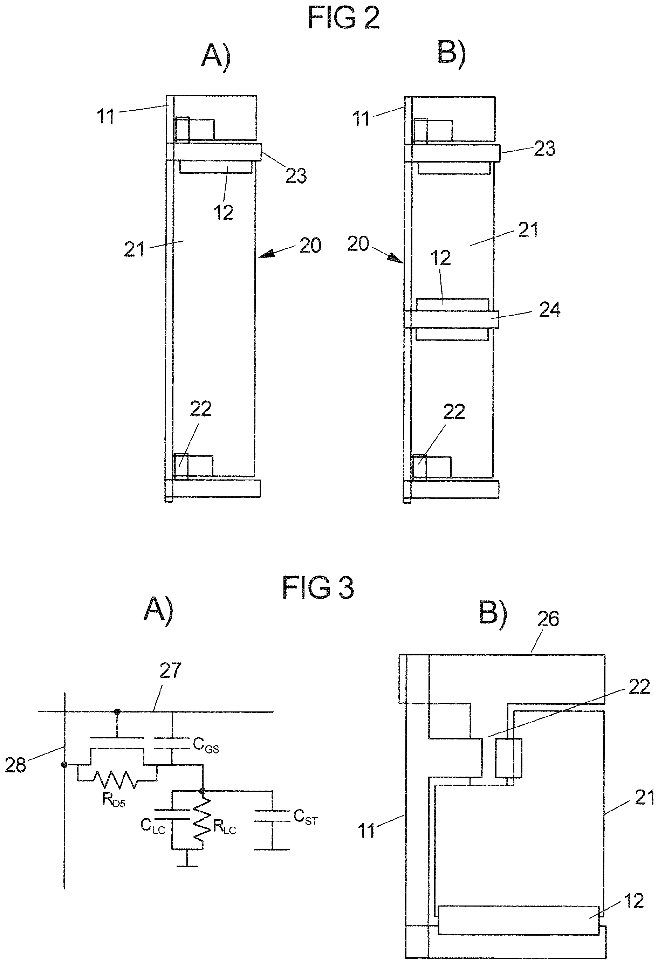

Vielfach wird ein weiterer Massedraht eingeführt, wodurch die Eigenschaften des Displays **20** verbessert werden **(Fig. 2)**. **Fig. 2A** zeigt einen Speicherkondensator **12**, der direkt an den nächsten CS-Draht **23** gekoppelt ist und/oder eine Verbindung zu einer extra Masseleitung **24** aufweist **(Fig. 2B)**.

Die Flächenabdeckung mit Elektroden **21** beträgt etwa 90 %, was bedeutet, dass nur etwa 10% der Fläche durch den TFT **22** sowie den Speicherkondensator **12** belegt sind. **Fig. 3A** veranschaulicht das Prinzip einer Einzelzelle mit parasitären Elementen (Gatekapazität CGs) und den elektrischen Eigenschaften des Flüssigkristallfilms (CLc und RLc), welche in physiologischen Anwendungen aber vernachlässigbar sind. Die typische Geometrie eines Pixels ist in **Fig. 3B** gezeigt

(Quelle: http://www.intechopen.com/source/html/11268/media/image16.jpg)

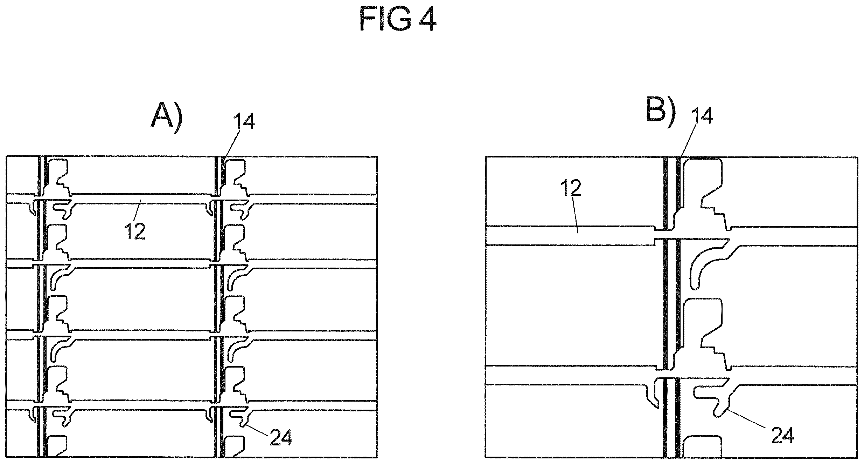

**Figur 4** zeigt die Geometrie der Backplane eines TFT-LCD in 20facher Vergrößerung (A) sowie Details des Transistors **24** und der Kondensatorstruktur in 40facher Vergrößerung. In dieser Darstellung ist der Kondensator **12** an einen Massedraht **14** gekoppelt.

## Ausführungsbeispiele

### Ausführungsbeispiel 1: Impedanzmessung mit Multielektrodenfeld

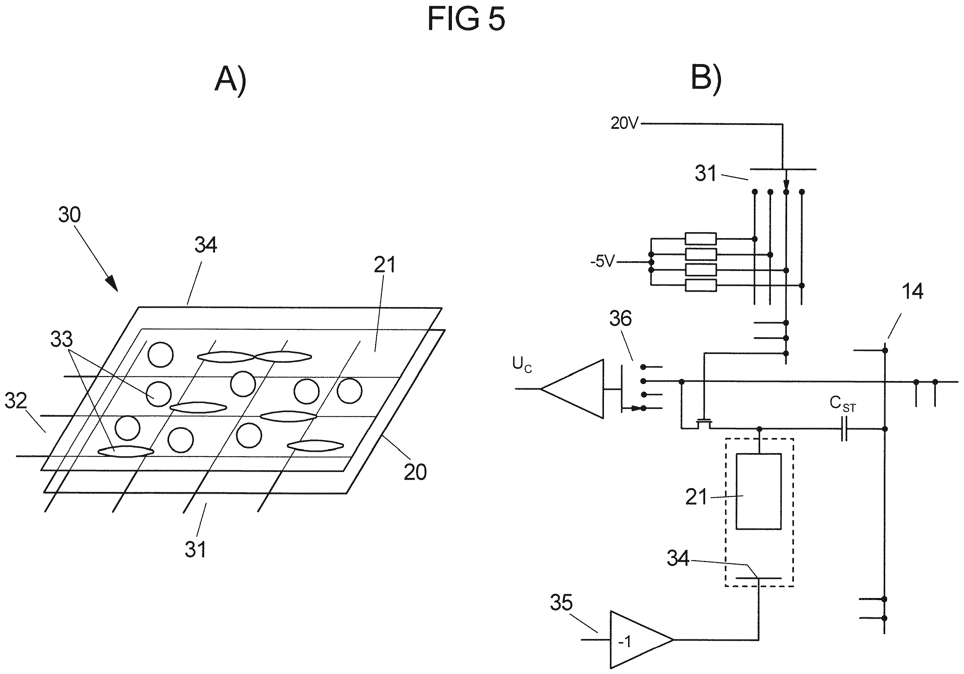

Für die Messung elektrischer Eigenschaften wird ein Pixel gemäß **Fig. 5** durch Auswahl von Spalte und Zeile kontaktiert. **Fig. 5** zeigt die prinzipielle Anordnung für die Verwendung eines kommerziellen Displays für die elektrische Charakterisierung biologischer Objekte. **Figur 5A** veranschaulicht die Messkammer **30** mit Backplane eines TFT-Displays **20** als Kontaktfläche mit einer distanten, den Elektrolyten kontaktierenden Gegenelektrode **34**. Die Zellen **33** adhärieren an den ITO-Elektroden **21**. Die ITO-Elektroden **21** sind in Reihen **31** und Spalten **32** angeordnet. **Figur 5B** veranschaulicht die Applikation eines Stimulus. Ein gemeinsamer Stimulus **35** wird an der Gegenelektrode **34** appliziert während die Spannung über den Speicherkondensatoren nach einer vordefinierten Zeit gemessen wird. Durch den direkten Zugang zu der selektierten Elektrode **21** können die Eigenschaften der adhärierten Zellen **33** praktisch mit jedem gängigem Verfahren bestimmt werden. Durch den Speicherkondensator werden dabei allerdings die hohen Frequenzanteile stark gedämpft. In einer praktischen Anordnung wird die Gegenelektrode **34** mit einem Multisinussignal beaufschlagt und der Strom durch die selektierte Elektrode **21** gemessen. Wird die Gegenelektrode **34** auf Massepotential gezogen, können durch die Zelle **33** generierte Ströme gemessen werden. Ein kurzer Stimulus mit höherer Spannung (>1V) an der Gegenelektrode **34** kann dabei eine aktive Antwort der Zelle **33** (Aktionspotential) auslösen. Durch Applikation eines noch stärkeren Stimulus **35** können die Zelleigenschaften durch Elektroporation beeinflusst werden. Durch Selektion anderer Elektroden **21** ist es auch möglich, das gesamte Elektrodenfeld anzusprechen.

### Ausführungsbeispiel 2: Parallele Messung

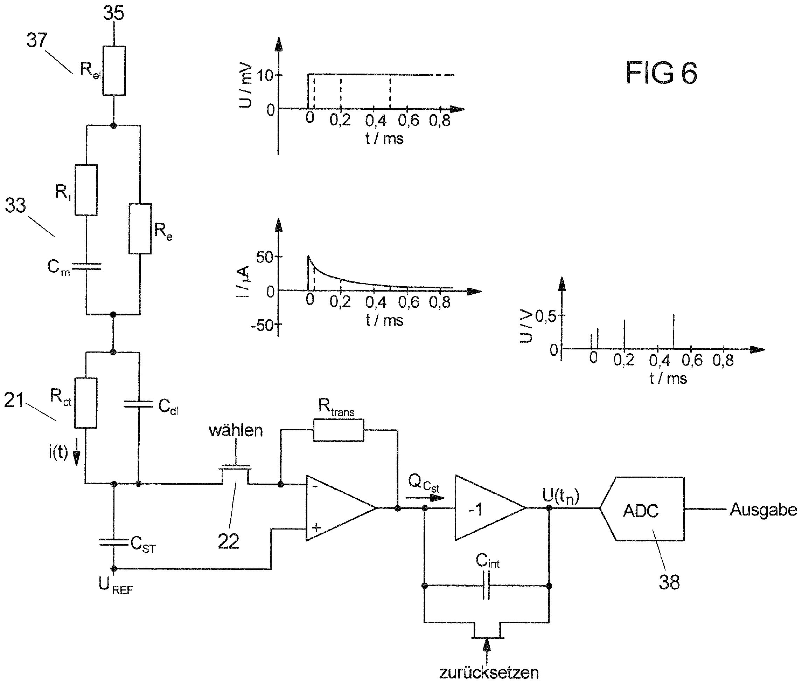

Für eine hohe Geschwindigkeit bei der Messung sowohl aktiver als auch passiver elektrischer Eigenschaften an allen Einzelelektroden müssen zunächst alle Elektroden deselektiert werden. Damit liegt das mit den Elektroden kontaktierte Objekt in Reihe mit dem Speicherkondensator gegen Masse (gemeinsamer CS-Draht). Durch die Applikation eines Spannungssprungs an der Gegenelektrode fließt entsprechend der Impedanz an dieser Elektrode ein Strom, welcher im Speicherkondensator integriert wird. Die Spannung über diesem Kondensator muss anschließend rechnerisch kompensiert werden. Durch Abtastung der Kondensatorspannung zu vorgegebenen Zeiten kann die individuelle Sprungantwort für alle Elektroden direkt gemessen werden, was jedoch technisch derzeit nicht für hochkanalige Systeme realisiert werden kann. Deshalb wird die Gegenelektrode zu den vorgegebenen Zeitpunkten hochohmig geschaltet, was dazu führt, dass die aktuelle Spannung über den Speicherkondensatoren gehalten wird. Anschließend werden alle Kondensatoren sequentiell über einen Ladungsspiegel entladen und die Ausgangsspannung digital umgesetzt. Diese Spannung entspricht der durch die Elektrode innerhalb der Zeit vom Spannungssprung bis zum Abschalten transportierten Ladung (Spannung $U(t_n)$ in Fig. 6). Danach werden alle Elektroden selektiert und die Gegenelektrode auf Massepotential gezogen. Nach Sperrung aller Kanäle wird der nächste Sprung ausgelöst und nach der nächsten vorgegebenen Zeit die Gegenelektrode wieder hochohmig geschaltet. Dieser Zyklus wird solange wiederholt, bis alle Werte für alle Abtastzeiten (zwischen 10 und 100) vorliegen.

**Figur 6** veranschaulicht das Prinzip der parallelen Messung. Die Darstellung erfolgt an einem einzelnen Kanal.

Zunächst ist der TFT hochohmig (keine Elektroden selektiert) und der Stimulus **35** (Sprung mit Abschaltung nach vorgegebener Zeit) sorgt für einen Stromfluss durch den Elektrolyten **37,** durch die Zellen **33** sowie durch die Elektroden **21** und damit durch den Kondensator, wodurch der Kondensator aufgeladen wird. Durch sequentielle Selektion aller Elektroden wird der Speicherkondensator **12** entladen und eine der vorherigen Ladung entsprechenden Spannung digitalisiert. Durch Wahl der Zeiten, in denen der Stimulus aktiv ist, kann das zu erfassende Frequenzband eingestellt werden. Für hohe Präzision bei geringem Datenvolumen werden exponentiell ansteigende Zeitintervalle genutzt. Die Datenauswertung erfolgt entweder direkt im Zeitbereich, wobei jeder Zeitkonstante ein Relaxationsmechanismus zugeordnet wird, oder im Frequenzbereich basierend auf Frequenzdispersionen. Für die Umrechnung in den Frequenzbereich ist dabei eine analytische Methode erforderlich, da gängige Herangehensweisen unter Verwendung von diskreter Fouriertransformation (z.B. Fast Fourier Transformation) äquidistant abgetastete Signale erfordern.

**Figur 7** veranschaulicht exponentiell ansteigende Zeitintervalle für die Integration des durch die Elektroden fließenden Stroms. Die aktiv-elektrischen Eigenschaften der Zellen können durch Integration der Ströme gemessen werden. Die Gegenelektrode liegt dabei entweder auf Masse oder einem vordefinierten Potential. Eine Auslesefrequenz von 60 Hz ist dabei möglich. Für schnelleres Auslesen oder Erfassung des zeitlichen Verhaltens des Signals können einzelne Elektroden selektiert werden. In diesem Fall ist die zweidimensionale Erfassung der Signale nicht möglich. Werden einzelne Elektroden selektiert und gegen Masse gezogen, kann ein überhöhter Stimulus (bis 16V) der Manipulation der an diesen Elektroden adhärenten Zellen genutzt werden.

**Ausführungsbeispiel 3: Integration des Signals mit gradueller Abtastung**

Es wird ein Spannungs- oder Stromsprung appliziert und die Antwort über einen Pufferverstärker an das Objekt angepasst. Während bei einer Antwort auf einen Spannungssprung ein niederohmiger Verstärker verwendet wird, wird bei der Antwort auf einen Stromsprung vorzugsweise ein hochohmiger Eingang gewählt. Durch einen Widerstand wird die Spannung in einen Strom gewandelt und dieser in der nächsten Stufe integriert, wobei durch einen zum Integrationskondensator parallel liegenden Transistor dieser direkt vor Sprungapplikation entladen wird. Damit wird ein definierter Ausgangspunkt erreicht. Der integrierte Strom wird zu definierten Zeiten mit exponentiell ansteigendem Abstand abgetastet und digital gewandelt. Durch die vorherige Integration wird damit das Problem des Aliasing bei niederer Abtastrate verhindert.

**Figur 8** zeigt die Integration des Stroms über einen langen Zeitraum von einer Sekunde oder mehr bei hinreichender Auflösung am Anfang der Integration mit Abtastintervallen von einigen Nanosekunden. Diese Art der Integration erfordert einerseits eine hohe Verstärkung, auf der anderen Seite wird eine hohe Spannung am Integrationskondensator **41** erzeugt. Für die Erweiterung des Dynamikbereichs auf mehr als 6 Größenordnungen wird ein logarithmischer Verstärker **42** zwischen Integrator und Abtaststufe geschaltet. Durch Nachschalten eines Vollweg-Präzisionsgleichrichters **40** kann auch die komplementäre Halbwelle zur Auswertung hinzugezogen werden, wodurch das Signal-Rausch-Verhältnis verbessert wird. Ebenso können auf diese Weise geringe Nichtlinearitäten ausgeglichen werden. Für eine hohe Präzision, besonders im Hinblick auf die Unsicherheiten bei der Erzeugung des Stimulus **35** wird das Anregungssignal in gleicher Weise behandelt und abgespeichert. Die Transformation in den Frequenzbereich, F(U(t)) bzw. F(I(t)), kann nicht einfach mittels der üblichen Algorithmen für die diskrete Fouriertransformation bewerkstelligt werden sondern bedarf einer analytischen Lösung wobei das Produkt aus Testfunktion und Sinus- bzw. Cosinusfunktionen partiell zwischen zwei Abtastpunkten integriert wird. Dabei können beliebige Frequenzlinien als ganzzahlige Vielfache des Reziproken der Periodendauer generiert werden. Wird dies sowohl für den Strom als auch die Spannung durchgeführt, kann die Impedanz direkt durch die Division Z = F(U(t)) / F(I(t)) bestimmt werden.

Als Beispiel wird die Berechnung der Fouriertransformierten einer einfachen Relaxation gezeigt. Die Spannung u bei einer einfachen Relaxation über einem Integrationskondensator sei gegeben durch eine Summe von Exponentialfunktionen. Erfolgt die Abtastung einer Halbwelle in wachsenden Abständen, so können jeweils Abschnitte der Kurve $u_i$ durch eine Exponentialfunktion dargestellt werden.

$$u_i = a_i e^{-t/\tau_1} + c_i$$

Theoretisch reichen drei Abtastpunkte i, in diesem Fall mit gleichem Abstand, um die 3 Werte a, t und c zu berechnen.

$$\tau_i = \frac{t_i - t_{i-1}}{ln\frac{(U_{i-1} - U_i)}{(U_i - U_{i+1})}} \qquad a_i = \frac{(U_{i-1} - U_i)}{e^{\frac{-t_{i-1}}{\tau_i}} - e^{\frac{-t_i}{\tau_i}}} \qquad c_i = U_{i-1} - a_i\, e^{\frac{t_{i-1}}{\tau_i}}$$

Das Fourierintegral (Koeffizienten $A_a$ und $A_b$) wird dann partiell ($A_{a,i}$ und $A_{b,i}$) für den Real- und Imaginärteil über alle Harmonischen n berechnet.

$$A_{a,n,i} = \int_{t_{i-1}}^{t_{i+1}} (a_i e^{\frac{-t}{\tau_i}} + c_i) \cos(n\omega_0 t)\, dt = \left[ \frac{a_i e^{\frac{-t}{\tau_i}}[\sin(nt)/\tau_i - n\cos(nt)]}{n^2 + 1/\tau_i^2} + \frac{c\sin(nt)}{n} \right]_{t_{i-1}}^{t_{i+1}}$$

$$A_{b,n,i} = \int_{t_{i-1}}^{t_{i+1}} (a_i e^{\frac{-t}{\tau_i}} + c_i) \sin(n\omega_0 t)\, dt = \left[ \frac{a_i e^{\frac{-t}{\tau_i}}[n\sin(nt) - \cos(nt)/\tau_i]}{n^2 + 1/\tau_i^2} + \frac{c\sin(nt)}{n} \right]_{t_{i-1}}^{t_{i+1}}$$

Wegen der angenommen Symmetrie der Halbwellen ergibt sich die Fouriertransformierte als:

$$\mathcal{F}\big(U(t)\big) = 2(A_a + jA_b)$$

Die Impedanz ist dann:

$$Z(j\omega) = \frac{\mathcal{F}\big(U(t)\big)}{\mathcal{F}\big(I(t)\big)}$$

Dieser Algorithmus ist an die Integration von Strom und Spannung gebunden. Ansonsten muss durch die Division durch die negativen Amplitudenkoeffizienten (-A) differenziert werden. Die Zeitkonstanten bleiben unbeeinflusst.

Neben dem hier gezeigten Algorithmus gibt es eine Reihe weitere Algorithmen, bei denen beispielsweise die Abtastpunkte an eine Summe von Funktionen angepasst werden und jeweils das rechnerische Integral über eine Halbwelle aufsummiert wird.

Bei allen Algorithmen muss zwingend zwischen dem eingeschwungenen Zustand (Rechteckwelle) und einer einzelnen Sprungfunktion unterschieden werden. Ebenso muss das Einschwingen im Zuge mehrerer Einzelsprünge rechnerisch ausgewertet werden.

Alternativ wird bei hoher Signalqualität (kein Überschwingen, kein Dachabfall) nur das abhängige Signal (Sprungantwort) direkt im Zeitbereich ausgewertet, wobei jeder Polarisationsmechanismus durch eine Zeitkonstante und eine Relaxationsamplitude charakterisiert wird. Nachteilig ist bei dieser Variante, dass für hohe Grenzfrequenzen ein sehr schneller ADC erforderlich ist. Bei Verwendung eines auf sukzessiver Approximation beruhenden ADC kann die Geschwindigkeit sofort nach dem Sprung durch den Verzicht auf die maximale Auflösung erhöht werden, während im Bereich großer Abtastintervalle die volle Auflösung erreicht wird. Dieses Vorgehen ist insofern gerechtfertigt, da sofort nach dem Sprung die Präzision der Abtastpunkte, aber weniger die ADC Auflösung die Genauigkeit beeinflusst, während im Bereich langsamer Änderungen die Genauigkeit durch die ADC-Auflösung dominiert wird. Alternativ kann in jeder Periode nur ein Abtastwert bestimmt werden, wodurch ein präziser, dafür aber langsamer ADC verwendet werden kann. Nachteilig sind hier die längere Messzeit und die höhere Störempfindlichkeit durch Jitter und Driften.

**Ausführungsbeispiel 4: Präzisionsgleichrichtung statt Abtastglied**

Die Anregung erfolgt mit einer Folge von Sprungfunktionen, bei denen ein Sprung mit komplementärer Polarität jeweils den Ladungsausgleich zum vorhergehenden Sprung schafft, wodurch besonders für elektrochemische Untersuchungen bzw. Messungen an biologischen Materialien wichtig ist. Nach Konditionierung des Signals mittels Transimpedanz und Integration erfolgt eine Halbwellen-Präzisionsgleichrichtung. Dabei steigt die Spannung über dem Ladekondensator so lange, wie der Ausgang des Integrators größer ist. Schaltet der Stimulus auf die komplementäre Phase, wird die Spannung gehalten. Damit erübrigt sich eine separate Abtastschaltung. Für die Generierung des Wertes an einem bestimmten Abtastpunkt wird die Zeit nach dem Sprung variiert. Wird diese Zeit monoton aufsteigend eingestellt, kann auf den Reset des Ladekondensators verzichtet werden.

**Figur 9** veranschaulicht die Verwendung einer Präzisionsgleichrichtung **44** anstatt eines Abtastglieds **43**. Für die Einstellung definierter Bedingungen sofort nach einem Sprung wird der Integrationskondensator **41** am Ende der komplementären Phase entladen. Alternativ kann die Entladung des Integrationskondensators **41** während der komplementären Phase auch überwacht werden und der nächste Sprung bei Erreichen von 0V angestoßen werden. Der Vorteil eines solchen Vorgehens ist der völlige Ladungsausgleich und damit die geringste Belastung des Messobjektes. Der Wert eines einzelnen Abtastpunktes wird jeweils am Beginn der komplementären Phase mittels ADC erfasst. Vorteilhaft gegenüber der in Ausführungsbeispiel 3 dargestellten Variante ist die höhere Präzision durch den Verzicht auf eine Abtaststufe **43,** in welcher der Einfluss von Schalttransienten durch höhere Ladeströme und damit durch einen höheren Stromverbrauch minimiert wird. Besonders vorteilhaft ist diese Schaltung für monotone Funktionen, da auf das Entladen des Ladekondensators nach jedem Sprung verzichtet werden kann, wodurch der Stromverbrauch erheblich sinkt.

**Ausführungsbeispiel 5: Parallele Zwischenspeicherung der Abtastwerte**

Bis zur Integration entspricht das Verfahren dem Ausführungsbeispiel 3. Für kontinuierliche Messungen mit maximaler Wiederholfrequenz reichen 10 Abtastpunkte je Dekade aus, wodurch bei 6 Dekaden (beispielweise 100 Hz-100 MHz) 60 Umsetzungen pro Messung vorgenommen werden. Im vorliegenden Fall würden bei konstanten Abtastintervallen 5 ms / 60 = 83 $\mu$s für eine Analog-Digitalwandlung zur Verfügung stehen, wofür stromsparende und hochauflösende Analog-Digital-Wandler (16 bit) in Frage kommen. Dies funktioniert aber nur, wenn die ersten sehr dicht abgetasteten Werte über einen Zeitraum von bis zu 100 $\mu$s zwischengespeichert werden können. Daher werden, gesteuert durch ein Schieberegister, welches vom logarithmisch eingestellten Abtasttakt geschoben wird, in einer Kette von Abtastgliedern die einzelnen Werte in individuellen Kondensatoren zwischengespeichert. Das Schieberegister wird dabei so programmiert, dass eine Kette von H in das vorher mit L beschriebene Register geschoben wird, wodurch gewährleistet ist, dass die Spannung auf den Kondensatoren bis zur Abtastung gehalten wird. Eventuell muss eine rechnerische Korrektur erfolgen. Durch einen Multiplexer werden diese dann an den ADC gelegt und umgesetzt. Hierbei bieten sich energiesparende RISC-Prozessoren mit bis zu 20 Analogeingängen an. **Figur 10** veranschaulicht eine entsprechende Schaltung zur parallelen Zwischenspeicherung der Abtastwerte. Ähnlich dem Ausführungsbeispiel 4 kann auch hier eine Kette von Präzisionsgleichrichtern **40** verwendet werden, wobei die Ausgänge des Schieberegisters **45** das Signal auf einen Pegel unter 0 Volt ziehen. Es ist auch nicht erforderlich, wie im gezeigten Beispiel, 60 Abtaststufen zu verwenden, wenn nach jeder AD-Umsetzung die entsprechende Stufe wieder freigegeben und erneut für die Abtastung verwendet wird. Dies lässt sich in einfacher Wiese durch ein Muster, welches in das Schieberegister **45** geschoben wird, sowie die entsprechende Ansteuerung des Multiplexers erreichen. Weiterhin können Abtastglieder auch zu Blöcken zusammengefasst werden und durch mehrere ADCs ausgelesen werden. Die Erzeugung der Abtastzeitlage kann durch ein rückgekoppeltes Schieberegister erfolgen, wodurch eine aufwändige Zeitlagensteuerung entfallen kann. in diesem Fall sind die Abtastzeiten zwar bekannt, lassen sich aber nicht beliebig legen, was für die Messung aber nur einen geringen Einfluss hat. Diese Variante ist besonders für batteriebetriebene Lösungen geeignet.

**Liste der Bezugszeichen**

| | |
|---|---|
| 10 | Schaltung der Backplane eines TFT Displays, |
| 11 | Data-Draht, |
| 12 | Speicherkondensator |
| 13 | Auswahldraht, Select-Draht |
| 14 | Common, Masse |
| 15 | Rot |
| 16 | Grün |
| 17 | Blau |
| 18 | Datenpulse |
| 19 | Auswahlpulse |
| 20 | TFT-Display (Ausschnitt), Multielektrodenfeld |
| 21 | Elektrode, Pixelelektrode, ITO-Elektrode |
| 22 | Thin-Film-Transistor (TFT) |
| 23 | CS-Draht |
| 24 | zusätzliche Masseleitung |
| 26 | Gate-Draht |
| 27 | Gate-Spannung |
| 28 | Datenspannung |
| 30 | Messkammer |
| 31 | Zeile |
| 32 | Spalte |

| 33 | Zellen, biologische Objekte, Testmaterial |
| 34 | Gegenelektrode |
| 35 | Stimulus |
| 36 | Datenauswahl |
| 37 | Elektrolyt |
| 38 | Analog-Digital-Wandler, ADC |
| 39 | Ausgang |
| 40, 44 | Präzisionsgleichrichter |
| 41 | Integrationskondensator |
| 42 | logarithmischer Vertstärker |
| 43 | Abtaststufe (S&H, sample & hold) |
| 45 | Schieberegister |

**Patentansprüche**

1. Vorrichtung zur elektrischen Charakterisierung und/oder elektrischen Manipulation biologischer Objekte (33), wobei die Vorrichtung ein in Spalten (32) und Zeilen (31) orientiertes Multielektrodenfeld (20) und eine Anordnung, insbesondere für eine Interface-Elektronik, aufweist;
das Multielektrodenfeld (20) und die Elektroden (21) transparent sind und jeder Elektrode (21) ein integrierendes Glied und ein Schalter zugeordnet ist, wobei das integrierende Glied so gestaltet ist, dass ein durch die jeweilige Elektrode fließender Antwortstrom partiell integrierbar ist;
die Elektroden (21) so gestaltet sind, dass an den Elektroden (21) biologische Objekte (33) adhärieren können;
durch Auswahl mindestens einer Elektrode (21) das Impedanzspektrum der an dieser mindestens einen Elektrode (21) adhärierten biologischen Objekte (33) messbar ist;
**dadurch gekennzeichnet, dass**
die Anordnung
ein Mittel zur Applikation eines Spannungs- oder Stromsprungs (35);
einen Pufferverstärker, zur Anpassung der Antwort auf den Spannungs- oder Stromsprungs an die Objekte (33);
einen Vollweg-Präzisionsgleichrichter (40), zur Heranziehung der komplementären Halbwelle zur Auswertung;
einen Integrationskondensator (41);
eine Abtaststufe (43) oder einen Präzisionsgleichrichter (44);
einen logarithmischen Verstärker (42), der zwischen Integrationskondensator (41) und Abtaststufe (43) oder Präzisionsgleichrichter (44) angeordnet ist;
und einen ADC (46), der nach der Abtaststufe (43) oder dem Präzisionsgleichrichter (44) angeordnet ist; aufweist.

2. Vorrichtung nach Anspruch 1, **dadurch gekennzeichnet, dass** das integrierende Glied ein an einer Elektrode (21) integrierter Speicherkondensator (12) ist und das der Schalter ein Transistor ist.

3. Vorrichtung nach Anspruch 1 oder 2, **dadurch gekennzeichnet, dass** die Elektroden (21) so gestaltet sind, dass das Impedanzspektrum durch Auswertung der Antwort auf einen Spannungs- oder Stromsprung messbar ist.

4. Vorrichtung nach einem der Ansprüche 1 bis 3, **dadurch gekennzeichnet dass** das Multielektrodenfeld (20) ITO-Elektroden als Einzelelektroden (21) aufweist.

5. Vorrichtung nach einem der Ansprüche 1 bis 3, **dadurch gekennzeichnet, dass** das Multielektrodenfeld (20) eine Kombination von Einzelelektroden (21) und LEDs aufweist.

6. Vorrichtung nach einem der Ansprüche 1 bis 5, **dadurch gekennzeichnet, dass** das Multielektrodenfeld (20) an eine Leiterplatte gebondet wird, die eine Interface-Elektronik trägt, oder direkt auf die Oberfläche eines transparenten Materials aufgebracht wird.

7. Vorrichtung nach einem der vorherigen Ansprüche, **dadurch gekennzeichnet, dass** die Anordnung anstelle der Abtaststufe (43) einen Präzisionsgleichrichter (44) aufweist.

8. Vorrichtung nach einem der vorherigen Ansprüche, **dadurch gekennzeichnet, dass** die Anordnung anstelle der Abtaststufe (43) oder des Präzisionsgleichrichters (44) eine Kette von Abtaststufen (43) und ein Schieberegister (45) oder eine Kette von Präzisionsgleichrichtern (44) und eine Schieberegister (45) aufweist.

9. Verfahren zur Detektion der passiv-elektrischen Eigenschaften eines biologischen Objekts mittels einer Vorrichtung nach einem der Ansprüche 1 bis 8, **dadurch gekennzeichnet, dass** das Verfahren die Schritte aufweist:

• Erzeugen eines sprunghaften Stromstimulus oder Spannungsstimulus (35), vorzugsweise einzeln an einer Elektrode oder gleichzeitig an allen Elektroden (21) eines Multielektrodenfeldes (20) einer Vorrichtung nach einem der Ansprüche 1 bis 6,
• Erfassung der individuellen Sprungantworten der einzelnen Elektroden (21) in Form des durch die Elektroden (21) fließenden Stroms, wobei eine zeitabhängige Messung des Stroms erfolgt, und
• Erfassung des Frequenzspektrums an mindestens einer einzelnen Elektrode (21).

10. Verfahren nach Anspruch 9, **dadurch gekennzeichnet, dass** der sprunghafte Stimulus (35) eine variable Länge besitzt, wodurch unterschiedliche Abtastpunkte der Stromantwort generiert werden.

11. Verfahren nach Anspruch oder 10, **dadurch gekennzeichnet, dass** der sprunghafte Stimulus (35) ein Rechteckpuls ist.

12. Verfahren nach Anspruch 9 oder 10 **dadurch gekennzeichnet, dass** Lage und Anzahl der Abtastpunkte durch die Länge des Stimulus (35) bestimmt werden und dass die Anzahl der Abtastpunkte vom Erzeugen des sprunghaften Stimulus bis zum Abschalten des Stimulus exponentiell ansteigt.

13. Verfahren nach einem der Ansprüche 9 bis 12, **dadurch gekennzeichnet, dass** das Verfahren weiterhin die Schritte aufweist:

• Ermittlung der mit interessierenden Objekten (33) adhärierten Elektroden (21); und
• selektives Auslesen dieser Elektroden (21) mit hoher Messwiederholrate.

14. Verfahren nach Anspruch 12, **dadurch gekennzeichnet, dass** die Impedanzspektren mit einem analytischen Algorithmus aus den Abtastpunkten berechnet werden.

15. Verfahren nach Anspruch 12 oder 14, **dadurch gekennzeichnet, dass** die Auswertung im Zeitbereich erfolgt, wobei jeder Zeitkonstante ein Polarisationsmechanismus zugeordnet wird.

16. Verfahren für eine breitbandige und stromsparende Bestimmung der elektrischen Impedanz eines Materials bzw. biologischen Objekts mittels einer Vorrichtung nach einem der Ansprüche 1 bis 8, wobei in dem Material eine sprunghafte Änderung eines elektrischen Stimulus erzeugt und die resultierende Antwort nach partieller Integration ausgewertet wird, **dadurch gekennzeichnet, dass** die Zeitlage für die Prozessierung der Sprungantwort sowie für die Generierung der Sprungfrage nach dem gleichen Takt erzeugt werden.

17. Verfahren zur Messung und/oder Beeinflussung der aktiv- elektrischen Eigenschaften adhärierter biologischer Objekte mittels einer Vorrichtung nach einem der Ansprüche 1 bis 8, **dadurch gekennzeichnet, dass** die Gegenelektrode (34) auf Massepotential gezogen wird und die durch die Zellen verursachten Ströme integriert werden.

18. Verfahren nach Anspruch 17, **dadurch gekennzeichnet, dass** durch statische Selektion mindestens einer Elektrode (21) des Multielektrodenfeldes (20) und kontinuierliche Aufzeichnung der Spannung über dem Ladekondensator und nach Erkennen von Elektroden mit erregbarer Membran eines biologischen Objekts Aktionspotentiale mit hoher Zeitauflösung gemessen werden.

19. Verfahren nach Anspruch 17, **dadurch gekennzeichnet, dass** das Verfahren weiterhin einen Schritt der Generierung eines Selektionsmusters für die einzelnen Elektroden (21) des Multielektrodenfeldes (20) aufweist.

20. Verfahren nach Anspruch 19, **dadurch gekennzeichnet, dass** durch Selektion aller Elektroden (21) ein gemeinsamer Stimulus (35) zur Induktion von Elektroporation oder einem Aktionspotential erzeugt wird.

**Claims**

1. Apparatus for electrical characterization and/or electrical manipulation of biological objects (33), wherein the apparatus comprises a multielectrode array (20) that is oriented along columns (32) and rows (31), and an arrangement, in particular for interface electronics; the multielectrode array (20) and the electrons (21) are transparent and each electrode (21) has assigned an integrating member and a switch, wherein the integrating member is designed in such a way that a response current flowing through the respective electrode is partly integrable; the electrodes (21) are designed in such a way that biological objects (33) able to adhere to the electrodes (21); the selection of at least one electrode (21) renders the impedance spectrum of the biological objects (33) that have adhered to this at least one electrode (21) measurable; **characterized in that** the arrangement comprises means for applying a voltage or current jump (35); a buffer amplifier for matching the response to the voltage or current jump to the objects (33); a full-wave precision rectifier (40) for using the complimentary half wave for evaluation purposes; an integration capacitor (41); a sampling stage (43) or a precision rectifier (44); a logarithmic amplifier (42) arranged between the integration capacitor (41) and the sampling stage (43) or precision rectifier (44) ;
and an ADC (46) arranged downstream of the sampling stage (43) or the precision rectifier (44).

2. Apparatus according to Claim 1, **characterized in that** the integrating member is a storage capacitor (12) integrated at the electrode (21) and **in that** the switch is a transistor.

3. Apparatus according to Claim 1 or 2, **characterized in that** the electrodes (21) are designed in such a way that the impedance spectrum is measurable by evaluating the response to a voltage or current jump.

4. Apparatus according to any one of Claims 1 to 3, **characterized in that** the multielectrode array (20) comprises ITO electrodes as individual electrodes (21).

5. Apparatus according to any one of Claims 1 to 3, **characterized in that** the multielectrode array (20) comprises a combination of individual electrodes (21) and LEDs.

6. Apparatus according to any one of Claims 1 to 5, **characterized in that** the multielectrode array (20) is bonded to a printed circuit board which carries interface electronics or applied directly to the surface of a transparent material.

7. Apparatus according to any one of the preceding claims, **characterized in that** the arrangement comprises a precision rectifier (44) in place of the sampling stage (43).

8. Apparatus according to any one of the preceding claims, **characterized in that** the arrangement comprises a chain of sampling stages (43) and a shift register (45) or a chain of precision rectifiers (44) and a shift register (45) in place of the sampling stage (43) or the precision rectifier (44).

9. Method for detecting the passive-electrical properties of a biological object by means of an apparatus according to any one of Claims 1 to 8, **characterized in that** the method includes the steps of:

   • producing a jump-like current stimulus or voltage stimulus (35), preferably individually at one electrode or simultaneously at all electrodes (21) of a multielectrode array (20) of an apparatus according to any one of Claims 1 to 6,
   • detecting the individual jump responses of the individual electrodes (21) in the form of the current flowing through the electrodes (21), a time-dependent measurement of the current taking place, and
   • detecting the frequency spectrum at at least one individual electrode (21).

10. Method according to Claim 9, **characterized in that** the jump-like stimulus (35) has a variable length, as a result of which different sampling points of the current response are generated.

11. Method according to Claim or 10, **characterized in that** the jump-like stimulus (35) is a rectangular pulse.

12. Method according to Claim 9 or 10, **characterized in that** the position and number of sampling points are determined by the length of the stimulus (35) and **in that** the number of sampling points increases exponentially from the production of the jump-like stimulus until the stimulus is switched off.

13. Method according to any one of Claims 9 to 12, **characterized in that** the method furthermore includes the steps of:

   • ascertaining the electrodes (21) to which objects (33) of interest adhere; and
   • selectively reading these electrodes (21) at a high measurement repetition rate.

**14.** Method according to Claim 12, **characterized in that** the impedance spectra are calculated from the sampling points using an analytical algorithm.

**15.** Method according to Claim 12 or 14, **characterized in that** the evaluation is implemented in the time domain, a polarization mechanism being assigned to each time constant.

**16.** Method for a broadband and current-saving determination of the electrical impedance of a material or biological object by means of an apparatus according to any one of Claims 1 to 8, wherein a jump-like change in an electrical stimulus is produced in the material and the resultant response is evaluated following partial integration, **characterized in that** the time position for processing the jump response and for generating the jump query are produced according to the same clock.

**17.** Method for measuring and/or influencing the active-electrical properties of adhered biological objects by means of an apparatus according to any one of Claims 1 to 8, **characterized in that** the counter electrode (34) is drawn to earth potential and the currents caused by the cells are integrated.

**18.** Method according to Claim 17, **characterized in that** action potentials are measured with a high time resolution by way of a static selection of at least one electrode (21) of the multielectrode array (20) and a continuous recording of the voltage across the charge capacitor and following the identification of electrodes with an excitable membrane of a biological object.

**19.** Method according to Claim 17, **characterized in that** the method furthermore includes a step of generating a selection pattern for the individual electrodes (21) of the multielectrode array (20).

**20.** Method according to Claim 19, **characterized in that** a common stimulus (35) for inducing electroporation or an action potential is produced by selecting all electrodes (21).

**Revendications**

**1.** Dispositif de caractérisation électrique et/ou de manipulation électrique d'objets biologiques (33), le dispositif comportant une zone à plusieurs électrodes (20) orientée en colonnes (32) et en rangées (31) et un ensemble, destiné en particulier à une électronique d'interface ; la zone à plusieurs électrodes (20) et les électrodes (21) étant transparentes et chaque électrode (21) étant associée à un élément d'intégration et à un commutateur, l'élément d'intégration étant conçu de telle sorte qu'un courant de réponse circulant à travers l'électrode respective puisse être partiellement intégré ;
les électrodes (21) étant conçues de manière que des objets biologiques (33) puissent adhérer aux électrodes (21) ;
la sélection d'au moins une électrode (21) permettant de mesurer le spectre d'impédance des objets biologiques (33) qui adhèrent à cette au moins une électrode (21) ;
**caractérisé en ce que**
l'ensemble comporte
un moyen d'application d'un saut de tension ou de courant (35) ; un amplificateur tampon destiné à adapter aux objets (33) la réponse au saut de tension ou de courant ;
un redresseur de précision pleine onde (40) destiné à utiliser la demi-onde complémentaire pour l'évaluation ;
un condensateur d'intégration (41) ;
un étage d'échantillonnage (43) ou un redresseur de précision (44) ;
un amplificateur logarithmique (42) qui est disposé entre le condensateur d'intégration (41) et l'étage d'échantillonnage (43) ou le redresseur de précision (44) ;
et un CAN (46) disposé en aval de l'étage d'échantillonnage (43) ou du redresseur de précision (44).

**2.** Dispositif selon la revendication 1, **caractérisé en ce que** l'élément d'intégration est un condensateur de mémorisation (12) intégré à une électrode (21) et **en ce que** le commutateur est un transistor.

**3.** Dispositif selon la revendication 1 ou 2, **caractérisé en ce que** les électrodes (21) sont conçues de manière que le spectre d'impédance puisse être mesuré par évaluation de la réponse à un saut de tension ou de courant.

**4.** Dispositif selon l'une des revendications 1 à 3, **caractérisé en ce que** la zone à plusieurs électrodes (20) comporte des électrodes ITO comme électrodes individuelles (21).

**5.** Dispositif selon l'une des revendications 1 à 3, **caractérisé en ce que** la zone à plusieurs électrodes (20) comporte une combinaison d'électrodes individuelles (21) et de LED.

**6.** Dispositif selon l'une des revendications 1 à 5, **caractérisé en ce que** la zone à plusieurs électrodes (20) est liée à un circuit imprimé qui porte une électronique d'interface, ou est appliquée directement sur la surface d'un matériau transparent.

**7.** Dispositif selon l'une des revendications précédentes, **caractérisé en ce que** l'ensemble comporte un redresseur de précision (44) à la place de l'étage d'échantillonnage (43).

**8.** Dispositif selon l'une des revendications précédentes, **caractérisé en ce que** l'ensemble comporte à la place de l'étage d'échantillonnage (43) ou du redresseur de précision (44) une chaîne d'étages d'échantillonnage (43) et un registre à décalage (45) ou une chaîne de redresseurs de précision (44) et un registre à décalage (45).

**9.** Procédé de détection des propriétés électriques passives d'un objet biologique au moyen d'un dispositif selon l'une des revendications 1 à 8, **caractérisé en ce que** le procédé comprend les étapes suivantes :

• générer un stimulus de courant ou stimulus de tension transitoire (35), de préférence individuellement sur une électrode ou simultanément sur toutes les électrodes (21) d'une zone à plusieurs électrodes (20) d'un dispositif selon l'une des revendications 1 à 6,
• acquérir les réponses transitoires individuelles des électrodes individuelles (21) sous la forme du courant circulant à travers les électrodes (21), une mesure du courant étant effectuée en fonction du temps, et
• acquérir le spectre de fréquences sur au moins une électrode individuelle (21).

**10.** Procédé selon la revendication 9, **caractérisé en ce que** le stimulus transitoire (35) a une longueur variable de façon à générer différents points d'échantillonnage de la réponse en courant.

**11.** Procédé selon la revendication ou 10, **caractérisé en ce que** le stimulus transitoire (35) est une impulsion rectangulaire.

**12.** Procédé selon la revendication 9 ou 10, **caractérisé en ce que** la position et le nombre de points d'échantillonnage sont déterminés par la longueur du stimulus (35) et **en ce que** le nombre de points d'échantillonnage augmente exponentiellement depuis la génération du stimulus transitoire jusqu'à l'arrêt du stimulus.

**13.** Procédé selon l'une des revendications 9 à 12, **caractérisé en ce que** le procédé comprend en outre les étapes suivantes :

• détecter les électrodes (21) qui adhèrent à des objets (33) dignes d'intérêt ; et
• lire sélectivement ces électrodes (21) à une fréquence de répétition de mesure élevée.

**14.** Procédé selon la revendication 12, **caractérisé en ce que** les spectres d'impédance sont calculés à partir des points d'échantillonnage avec un algorithme analytique.

**15.** Procédé selon la revendication 12 ou 14, **caractérisé en ce que** l'évaluation est effectuée dans le domaine temporel, un mécanisme de polarisation étant associé à chaque constante de temps.

**16.** Procédé de détermination à large bande et à faible consommation de courant de l'impédance électrique d'un matériau ou d'un objet biologique au moyen d'un dispositif selon l'une des revendications 1 à 8, une variation transitoire d'un stimulus électrique étant générée dans le matériau et la réponse résultante étant évaluée après intégration partielle, **caractérisé en ce que** l'intervalle de temps pour traiter la réponse transitoire et pour générer l'interrogation transitoire est généré à la même fréquence d'horloge.

**17.** Procédé de mesure des propriétés électriques actives d'objets biologiques qui adhèrent et/ou d'influence sur celles-ci au moyen d'un dispositif selon l'une des revendications 1 à 8, **caractérisé en ce que** la contre-électrode (34) est portée au potentiel de masse et les courants provoqués par les cellules sont intégrés.

**18.** Procédé selon la revendication 17,
**caractérisé en ce que** des potentiels d'action sont mesurés avec une résolution temporelle élevée par une sélection statique d'au moins une électrode (21) de la zone à plusieurs électrodes (20) et par l'acquisition continue de la tension aux bornes du condensateur de charge et après détection d'électrodes avec une membrane excitable d'un objet biologique.

19. Procédé selon la revendication 17, **caractérisé en ce que** le procédé comprend en outre une étape de génération d'un motif de sélection des électrodes individuelles (21) de la zone à plusieurs électrodes (20).

20. Procédé selon la revendication 19, **caractérisé en ce qu'**un stimulus commun (35) destiné à induire l'électroporation ou un potentiel d'action est généré par sélection de toutes les électrodes (21).

FIG 1

# FIG 2

A)

B)

# FIG 3

A)

B)

# FIG 4

A)

14

12

24

B)

14

12

24

EP 3 642 618 B1

# FIG 5

A)

B)

EP 3 642 618 B1

FIG 6

FIG 7

FIG 8

FIG 9

FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006011345 A1
- WO 2008072166 A1
- WO 2008072135 A2
- US 20170038282 A1
- US 2004152067 A

**In der Beschreibung aufgeführte Nicht-Patentliteratur**

- **GIAEVER I. ; KEESE C.R.** Monitoring fibroblast behavior in tissue culture with an applied electric field. *Proc Natl Acad Sci USA,* 1984, vol. 81, 3761-3764
- **GIAEVER I. ; KEESE C.R.** A morphological biosensor for mammalian cells. *Nature,* 1993, vol. 366, 591-592
- **RÄDLER, U ; WEGENER, J.** Impedanzbasiertes Screening adhärenter Zellen. *BIOspektrum,* 2009, vol. 15, 535-537
- **PLIQUETT, U.** Time-domain based impedance measurement: strengths and drawbacks. XV Int. Conf. on Electrical Bio-Impedance & XIV Conf. on Electrical Impedance Tomography. *IOP Publishing Journal of Physics: Conference Series,* 2013, vol. 434, 012092
- **PLIQUETT, U.** Adaptive signal sampling for high throughput, broadband impedance spectroscopy. *International Journal of Bioelectromagnetism,* 2015, vol. 17 (1), 20-25